**Europäisches Patentamt**

**European Patent Office**

**Office européen des brevets**

(19)

(11) Publication number: **0 054 273**

**A1**

(12) # EUROPEAN PATENT APPLICATION

(21) Application number: **81110358.9**

(22) Date of filing: **11.12.81**

(51) Int. Cl.³: **A 61 B 3/00**
**A 61 B 3/10**

(30) Priority: **12.12.80 US 215733**
**12.12.80 US 215734**

(43) Date of publication of application:
**23.06.82 Bulletin 82/25**

(84) Designated Contracting States:
**AT BE CH DE FR GB IT LI LU NL SE**

(71) Applicant: **Coherent, Inc.**
**3210 Porter Drive**
**Palo Alto, Cal. 94303(US)**

(72) Inventor: **Munnerlyn, Charles R.**
**1360 Bedford Avenue**
**Sunnyvale, Cal.(US)**

(72) Inventor: **Glockler, Hermann J.**
**10395 Melissa Court**
**Cupertino, Cal.(US)**

(72) Inventor: **Clapham, Terrance N.**
**2884 Mayglen Way**
**San Jose, Cal.(US)**

(74) Representative: **Reinländer & Bernhardt Patentanwälte**
**Orthstrasse 12**
**D-8000 München 60(DE)**

(54) Patient alignment system.

(57) A system for aligning an auto refractor (10) and the eyes of the patient having a movable chin rest (12) to move in the vertical direction (Y) and a movable forehead brace (14) to adjust the vertex distance (Z). In order to analyze the left and right eyes the instrument (22) is movable in a direction (X) perpendicular to the plane defined by the vertical direction (Y) and the vertex distance (Z). In addition, when the eyes are at a fixed distance from the instrument (22), the system has means (16, 21) for moving the eyes of the patient and means (32, 34, 35, 38) substantially in the same imaging plane as the imaging plane of the eyes for indicating when the eyes are at the requisite fixed distance.

FIG. 1.

EP 0 054 273 A1

## Description
## Patient Alignment System

### Technical Field

The present invention relates to a system for aligning an anatomical portion of a patient with an instrument, and more particularly, to an alignment system adapted for use with an auto refractor.

### Background Art

Auto refractors, i.e., instruments that determine the refractive power of an eye of a patient (in other words whether or not the patient needs a pair of corrective lenses), are well known in the art. Units, such as Dioptron, Dioptron II, or Dioptron Ultima (Dioptron is a registered trademark of Coherent Radiation, Inc.), have been manufactured and marketed by Coherent, Inc. In these units, the patient's chin is placed on a chin rest in a manner similar to that used in a slit lamp or other ophthalmic instruments. The patient's forehead is braced against a forehead brace. Although the chin rest may be moved slightly up and down to accommodate different head sizes of different patients, once the patient's head is in place it is held stationary. Only the instrument is then moved in any one of three directions to align with the eyes of the patient. Because the instrument must be able to move in three mutually orthogonal directions, the alignment system to align the instrument with the patient's eyes has been complex and unwieldy. In addition, since each eye must be maintained at a fixed distance from the instrument during the analysis, the alignment system has been prone to error due to its complexity.

In some ophthalmic instruments, where it is also critical to maintain the eye at a fixed distance from the instrument during the analysis, marker means, such as cross-hair, is used. When the image of the eye, as seen through a viewing port is aligned or impinges the image of the marker means, the eye is then at the requisite distance. In these instruments, however, the image of the marker means is in a plane different from the image of the eye. Thus, parallax occurs. As a result, inaccuracy in aligning the image of the eye with the image of the marker means, which means inaccuracy in adjustment to the requisite distance, is produced.

Disclosure of the Invention

Therefore, in accordance with the present invention, an alignment system for aligning an anatomical portion of a patient with an instrument along a path, has a means for moving the anatomical portion in a first direction which is adapted to change the distance of separation between the instrument and the anatomical portion along the path. The system further comprises means for moving the instrument in a second direction which is substantially perpendicular to the first direction. Finally, means is provided for adjusting the anatomical portion in a third direction which is a direction substantially perpendicular to a plane defined by the first and second directions.

Further, in accordance with the present invention, an alignment system for aligning an anatomical portion of a patient with an instrument along a path, and wherein the instrument must be at a fixed distance from the anatomical portion during the analysis thereof by the instrument, has a means for adjusting the distance of separation along a direction parallel to the path between the anatomical portion and the instrument. Viewing means is provided to view the anatomical portion along a trajectory which is aligned to impinge the anatomical portion in a direction substantially perpendicular to the path. Marking means is in the field of view of the viewing means, and is positioned such that as seen through the viewing means it is in the same imaging plane as the anatomical portion, and when the image of the anatomical portion is superimposed on the image of the marking means, the anatomical portion is at the fixed distance.

Brief Description of the Drawings

Figure 1 is a perspective, partially cut-away view, of an instrument containing the alignment system of the present invention.

Figure 2 shows an image of an eye of a patient as viewed through one of the viewing ports of the instrument of Figure 1 when the eye is at the requisite vertex distance.

Figure 3 is a front view, partially cut away, showing the apparatus to generate the light bar marking that is used to determine the requisite vertex distance.

Figure 4 is a view of an eye of the patient as seen through another viewing port.

Figure 5 is a top view, partially cut away of the instrument shown in Figure 1, showing the alignment optics for the image as seen through the viewing port of Figure 4.

Figure 6 shows a rack and pinion gear mechanism for moving the forehead brace used in the instrument shown in Figure 1.

Figure 7 is yet another rack and pinion mechanism to move the chin rest used in the instrument shown in Figure 1.

Figure 8 is a screw mechanism used to move the instrument shown in Figure 1.

## Detailed Description of the Invention

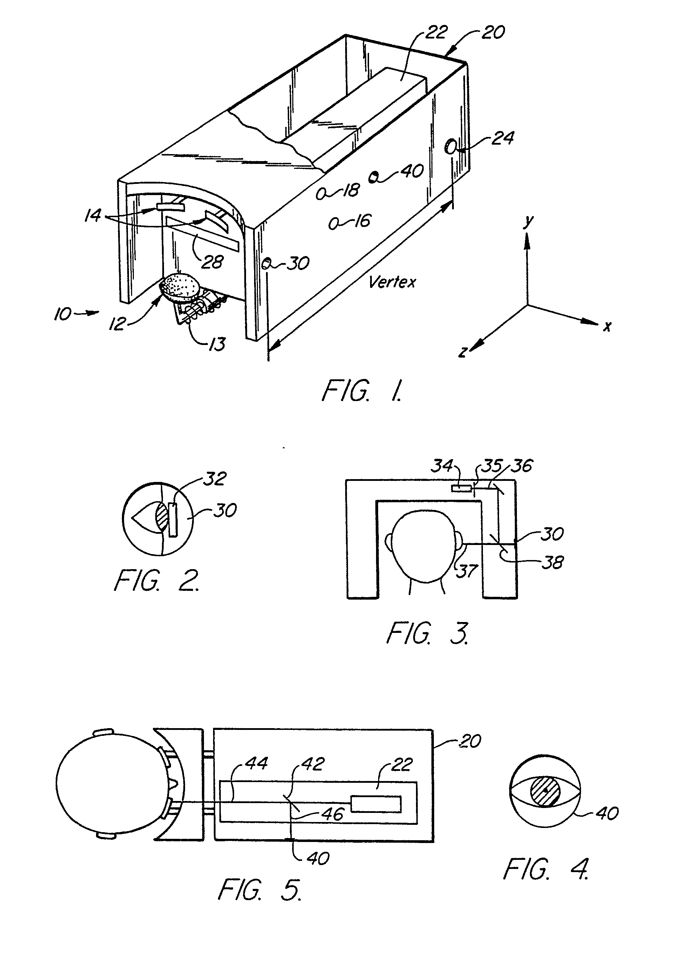

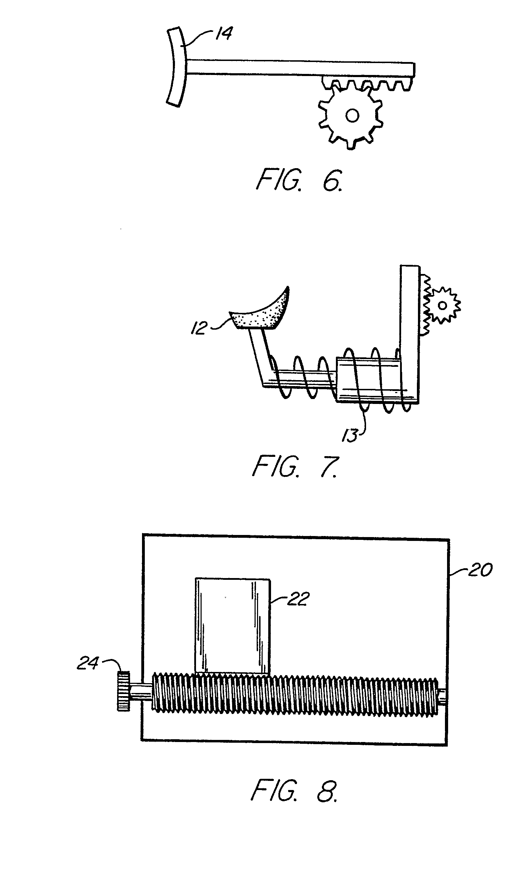

Referring to Figure 1 there is shown an auto refractor 10 incorporating the alignment system of the present invention. The auto refractor 10 comprises a chin rest 12 on which the patient's chin is placed. The patient's forehead is placed against the forehead brace 14. In this manner, the patient's head is held during the alignment process. Typically, the auto refractor 10 is placed on a table (not shown), while the patient sits in a chair (also not shown). The chin rest 12 may be moved up and down, along the Y direction by, for example, a rack and pinion gear arrangement such as that shown in Figure 7. A first knob 16 is adapted to adjust the position of the chin rest 12. A spring 13 urges the chin rest 12 in the Z direction. In the absence of a patient, the spring 13 urges the chin rest 12 away from the housing 20. A second knob 18 is connected to another rack and pinion gear mechanism which is adapted to move the forehead brace 14. By adjusting the second knob 18 the forehead brace 14 and the forehead of the patient may be moved along the Z direction. The instrument 22 adapted to analyze the refractory power of the patient's eye is contained in housing 20. The instrument 22 is adapted to be moved along the X direction by a third knob 24. The third knob 24 is connected through a screw thread arrangement. The instrument 22 is adapted to analyze the refractory power of the patient's eyes along a path, parallel to the Z direction through the housing opening 28 and to impinge the patient's eyes. The three directions, X, Y, and Z are mutually orthogonal directions. In general, X and Z are horizontal directions while Y is a vertical direction. Thus through the appropriate manipulations of first, second, and third knobs 16, 18 and 24 respectively the instrument 22 may be aligned with any portion of the anatomy of the patient. It should be clear from the foregoing that any mechanical linkage may be used to move the forehead brace 14, the chin rest 12, and the instrument 22.

In the operation of the auto refractor 10, the patient is first comfortably positioned such that the patient's chin rests on the chin rest 12. The patient then moves the chin and the chin rest 12 toward the housing 20, compressing the spring 13 until the patient's forehead is against the forehead brace 14. Next, the operator of the auto refractor 10 looks through the first viewing port 30. The operator would see a bar of light 32. Through the adjustment of the first and second knobs 16 and 18 respectively the patient's forehead and chin are moved until the cornea of the eye of the patient as seen through the first viewing port 30 is superimposed with the center of the bar of light 32. This is shown in Figure 2. When this has occurred, the patient's eye is positioned at a requisite fixed distance (called vertex distance) from the instrument 22 along the Z direction. The adjustment of the second knob 18 moves only the forehead brace 14 in the Z direction. However, since the chin is on a chin rest 12 which is biased in the Z direction by the spring 13, any movement of the forehead in the Z direction also results in the chin rest 12 moving in that Z direction. The bar of light 32 is merely a marking means to indicate when the cornea of the eye of the patient is at the requisite fixed distance from the instrument 22. Other marking means, such as a beam of light having a cross hair pattern may be suitably employed. The bar of light 32 is generated by a lamp 34 having a slit 35 therein. The beam generated by the lamp 34 travels along the path 36 and impinges a beam splitter 38. The bar of light 32 is reflected from the beam splitter 38 and is passed through the first viewing port 30. The image of the eye is also passed through the beam splitter 38, along path 37 and is super positioned on the image of the bar of light 32 through the first viewing port 30. The first viewing port 30 is aligned in a direction which is substantially perpendicular to the Z direction which is the direction parallel to the path from the instrument 22 to the patient's eye. The distance from the first viewing port 30 to the slit 35 along the path 36 is substantially the same as the distance from the first viewing port 30 to the eye along the path 37. Thus, the images of the eye and of the bar of light 32 are substantially in the same image plane, albeit the image of the bar of light 32 is a virtual image. By having the image of the bar of light 32 substantially in the same imaging plane as the image of the eye parallax is reduced. Accuracy in focussing is increased. As previously stated, when the second nob 18 is moved, it adjusts the forehead brace 14 which changes the distance of the eye from the instrument 22 along the Z axis.

The alignment along the X and Y directions then proceeds in the following manner. The operator looks through a second viewing port 40. From the second viewing port 40 the operator sees the eye directly head on, i.e., along the Z axis in the same manner as the instrument 22 sees the eye. When the eye of the patient is centered within the second viewing port 40, then the eye is correctly aligned for analysis by the instrument 22. Through the adjustment of the first and third knobs 16 and 24 respectively the patient's chin may be raised or lowered in the Y direction, while the instrument may be moved in the X direction. Thus, through the combination of the movement along the X and Y directions the relative position of the patient's head and the instrument 22 may be moved until the eye of the patient is totally within the view of the second port 40. In actuality, the second viewing port 40 views the eye along the same path as the path used for analysis by the instrument 22. The instrument 22 analyzes the eye along the path 44. A mirror 42 is placed in the path 44 at 45° with respect thereto. The path 44 being from the eye is reflected onto the mirror 42 and on to the path 46 out through the second viewing port 40. Thus, as seen through the second viewing port 40, the image of the eye is along the same path 44 as that ultimately seen by the instrument 22. During the operation of the instrument 22, the mirror 42 is swung out of the way by an electrically activated solenoid, thereby permitting the image of the eye as seen along path 44 to enter into the instrument 22.

Finally, once the eye of the patient has been aligned along Z, Y and X directions, the operator proceeds with the examination of the refractory power of one of the eyes. Such analysis by the instrument 22 is well known in the art and will not be discussed herein. Once the examination for that eye is finished, third knob 24 is activated moving the instrument 22 in the X direction to the other eye, while the patient remains stationary. The operator may choose to make sure that the second eye is correctly aligned with respect to the instrument 22 by checking the first viewing port and 30 and second viewing port 40 and using the procedure as set forth hereinabove. The refraction of the second eye may then be determined.

There are many advantages to the alignment system of the present invention. First and foremost is the simplicity of operation. The simplicity of the system further offers accuracy in the alignment process. Moreover, the system is easier and less costly to manufacture. Finally, reduction in parallax is accomplished by positioning the image of the marking means substantially in the same imaging plane as the image of the eye. Clearly, the instrument has other uses and is not limited to its application in an auto refractor.

.C.L A.I M S

1. A patient alignment system for aligning an anatomical portion of said patient with an instrument (22) along a path (44),said system comprising:

means (14, 18) for moving said anatomical protion in a first direction (Z), said moving means (14, 18) adapted to change .the distance of separatian beween said instrument (22) and said portion along said path (44);

means (24) for moving said instrument (22) in a second direction (X), said moving means (24) adapted to move said instrument (22) in a direction (X) substantially perpendicular to said first direction (Z); and

means (12, 16) for adjusting said anatomical portion in a third direction (Y), said adjusting means (12, 16) adapted to move said anatomical portion in a direction (Y) substantially perpendicular to a plane defined by said first (Z) and second directions (X).

2. The system of Claim 1 further comprising means (40, 42) for viewing said anatomical portion along said first direction (Z, 44).

3. The system of Claim 2 wherein said viewing means (40, 42) is aligned to view said anatomical portion along the same path (44) as the path of said instrument (22).

4. The system of Claim 3 wherein said adjusting means (12, 16) further comprises spring means (13) for biasing said adjusting means (12, 16) along said first direction (Z)

5. The system of Claim 1 wherein said first direction (Z) is substantially horizontal.

6. The system of Claim 5 wherein said second direction (X) is substantially horizontal.

7. The system of Calim 6 wherein said third direction (Z) is substantially vertical.

8. A patient alignment system for aligning an anatomical portion of said patient with an instrument (22) along a path (44), wherein said instrument (22) must be at a fixed distance from said portion during the use of said instrument (22), said system comprising:

   means (14, 18) for adjusting the distance of separation along a direction (Z) parallel to said path (44) between said anatomical portion and said instrument (22);

   means (30) for viewing said anatomical portion;
   said viewing means aligned to view said anatomical portion along a trajectory (37);
   at least a portion (37) of said trajectory is in a direction substantially perpendicular to said path (44);
   marking means (32) in the field of view of said viewing means (30); and
   said marking means (32) positioned such that, as seen through said viewing means (30), it is in the same imaging plane as said anatomical portion, and such that when the image of said anatomical portion as seen through said viewing means (30) is superimposed on said marking means (32), said anatomical portion is at said fixed distance.

9. The system of Claim 8 wherein said trajectory (37) is aligned to impinge said anatomical portion in a direction substantially perpendicular to said path (44).

10. The system of Claim 9 wherein said marking means is an illuminated bar of light (32).

11. The system of Claim 10 further comprising:

   means (34) for generating a beam of light;
   slit means (35);
   said beam aligned to pass through said slit means (35);
   beam splitter means (38); and
   said beam aligned to impinge said beam splitter means (38) and to reflect therefrom through said viewing means (30) to produce said illuminated bar of light (32).

12. The system of Claim 11 wherein the distance from said viewing means (30) to said slit means (35) along said beam path is substantially the same as the distance from said viewing means (30) to said anatomical portion along said trajectory (37).

13. The system of Claim 12 wherein said trajectory (37) is aligned to pass through said beam splitter means (38).

14. The system of Claim 1 or 8 wherein said adjusting means (14, 18) is adapted to move the forehead.

15. The system of Calim 1 or 8 wherein said anatomical portion is the eye.

0054273

FIG. 1.

FIG. 2.

FIG. 3.

FIG. 5.

FIG. 4.

212

FIG. 6.

FIG. 7.

FIG. 8.

European Patent
Office

**EUROPEAN SEARCH REPORT**

| | **DOCUMENTS CONSIDERED TO BE RELEVANT** | | CLASSIFICATION OF THE APPLICATION (Int. Cl. 3) |
|---|---|---|---|
| Category | Citation of document with indication, where appropriate, of relevant passages | Relevant to claim | |
| Y | US - A - 2 081 969 (C.C. ALLEN et al.)<br><br>* Page 2, left-hand column, line 73 - right-hand column, line 21; page 3, left-hand column, lines 17-36; figures 1,2 * | 1-3,5-7,15 | A 61 B 3/00<br>3/10 |
| Y | US - A - 3 797 921 (L.G. KILMER et al.)<br><br>* Abstract; column 6, line 25 - column 7, line 29; figure 3,8-10 * | 1-3,5,7,14,15 | |
| | | | TECHNICAL FIELDS SEARCHED (Int.Cl. 3) |
| A | US - A - 2 162 617 (C. HUMMON)<br><br>* Page 1, left-hand column, line 50 - right-hand column, line 13; page 2, left-hand column, line 18 - right-hand column, line 26; figures 1-4 * | 1,5,7,14,15 | A 61 B 3/00<br>3/10 |
| A | US - A - 2 635 502 (J.M. RICHARDS)<br><br>* Column 5, line 10 - column 6, line 11; figures 1,2,4 * | 1,5-7,14,15 | |
| | | | CATEGORY OF CITED DOCUMENTS |
| A | GB - A - 1 115 275 (R. RODENSTOCK)<br><br>* Page 1, lines 10-15, lines 24-35 and lines 54-70; figure 1 * | 8-10,15 | X: particularly relevant if taken alone<br>Y: particularly relevant if combined with another document of the same category<br>A: technological background<br>O: non-written disclosure<br>P: intermediate document<br>T: theory or principle underlying the invention<br>E: earlier patent document, but published on, or after the filing date<br>D: document cited in the application<br>L: document cited for other reasons |
| A | DE - A - 2 019 720 (AMERICAN OPTICAL CORP.)<br><br>* Page 2, lines 5-12; page 2, line 29- page 3, line 13; page 6, lines 4-16; page 7, lines 5-9;    ./. | 8,9,14,15 | |

| | The present search report has been drawn up for all claims | &: member of the same patent family, corresponding document |
|---|---|---|
| Place of search<br>The Hague | Date of completion of the search<br>17-03-1982 | Examiner<br>RIEB |

EPO Form 1503.1 08.78

| | DOCUMENTS CONSIDERED TO BE RELEVANT | | CLASSIFICATION OF THE APPLICATION (Int. Cl.³) |
|---|---|---|---|
| Category | Citation of document with indication, where appropriate, of relevant passages | Relevant to claim | |
| | page 8, lines ¹-10; figure 3 * | | |
| | ---- | | |

TECHNICAL FIELDS
SEARCHED (Int. Cl.³)

EPO Form 1503.2 06.78